# FASCICULE DE BREVET EUROPEEN

(11) **EP 3 313 452 B1**
(45) Date de publication et mention de la délivrance du brevet: **04.09.2019**
(21) Numéro de dépôt: 16736898.4
(22) Date de dépôt: 15.06.2016
(51) Int. Cl.: A61K 47/36, A61K 8/73, A61K 31/728, C08J 3/075, C08J 3/24

(54) **PROCEDE DE PREPARATION D'UN HYDROGEL RETICULE INJECTABLE; HYDROGEL OBTENU; UTILISATION DE L'HYDROGEL OBTENU**
VERFAHREN ZUR HERSTELLUNG EINES INJIZIERBAREN VERNETZTEN HYDROGELS, ERHALTENES HYDROGEL UND VERWENDUNG DES ERHALTENEN HYDROGELS
METHOD FOR PREPARING AN INJECTABLE CROSS-LINKED HYDROGEL, HYDROGEL OBTAINED; AND USE OF THE OBTAINED HYDROGEL

(30) Priorité: 24.06.2015 FR 1501323
(43) Date de publication de la demande: 02.05.2018
(73) Titulaire: Kylane Laboratoires SA, Plan-Les-Ouates 1228 (CH)
(72) Inventeur: TAUZIN, Bénédicte Vincente, 74250 Bogeve (FR)
(74) Mandataire: reuteler & cie SA
(86) Numéro de dépôt international: PCT/FR2016/000096
(87) Numéro de publication internationale: WO 2016/207496

(56) Documents cités:
- WO-A1-94/25078
- WO-A1-2014/198406
- WO-A2-2014/173941

## Description

La présente invention a pour objet :
- un procédé de préparation d'un hydrogel injectable à base d'acide hyaluronique réticulé, ou l'un de ses sels, et éventuellement d'autres polymères biocompatibles
- un hydrogel obtenu selon ledit procédé de préparation
- l'utilisation de l'hydrogel obtenu selon ledit procédé de préparation dans les domaines de l'esthétique et de la médecine

L'acide hyaluronique est un polysaccharide formé par la répétition d'une unité disaccharidique composée d'acide D-glucuronique et de N-acétylglucosamine. Sa structure est linéaire et sans spécificité d'espèce. L'acide hyaluronique est largement distribué dans les organismes vivants humains et animaux, dans lesquels il joue de nombreuses fonctions biologiques comme par exemple le contrôle du taux d'hydratation ou le maintien de la viscoélasticité de fluides ou de tissus. On le retrouve notamment en concentration élevée dans le liquide synovial, le corps vitreux de l'oeil et dans le derme. Un être humain de 70 kg possède environ 15 g d'acide hyaluronique dont la moitié est contenue dans la peau et cette quantité diminue avec le vieillissement.

Les hydrogels d'acide hyaluronique sont connus et utilisés dans de larges domaines de l'esthétique et de la médecine depuis de nombreuses années. Ces gels sont, notamment, couramment injectés :
- dans les yeux, au cours de chirurgies ophtalmologiques, afin de maintenir l'espace intra-oculaire et protéger les tissus de l'oeil
- dans les articulations, en cas d'arthrose, pour supplémenter le liquide synovial déficient et restaurer temporairement les propriétés chondroprotectrices dudit liquide biologique
- dans ou sous la peau, afin de combler des rides ou d'augmenter le volume de certaines zones du visage ou du corps

L'acide hyaluronique possède une demi-vie faible dans les organismes vivants (moins de 1 semaine).

Dans de nombreuses applications en esthétique et en médecine, il est injecté chez les patients sous sa forme native, c'est-à-dire qu'il n'est pas réticulé et/ou modifié chimiquement.

Pour d'autres applications, il est administré chez les patients sous une forme stabilisée par réticulation. La réticulation permet de considérablement augmenter la durée de vie (encore appelée rémanence) de l'acide hyaluronique *in vivo,* mais elle permet également de modifier ses propriétés mécaniques et rhéologiques en le rendant notamment plus élastique, ce qui permet alors d'accroître sa capacité à créer du volume une fois injecté dans les tissus souhaités. Ainsi, grâce à cette modification par réticulation, un hydrogel à base d'acide hyaluronique réticulé a par exemple la capacité de combler des rides sur une période de plusieurs mois.

Compte tenu du rôle clé que joue l'acide hyaluronique réticulé dans les domaines-de l'esthétique et de la médecine, l'homme de l'art cherche constamment à améliorer la performance de cette molécule en lui octroyant de meilleures caractéristiques biomécaniques et/ou de rémanence, tout en lui conservant un très haut niveau de sécurité.

Les demandes WO2014/173941 et WO2014/198406 proposent une procédé de fabrication d'un gel aqueux injectable comprenant de l'acide hyaluronique réticulé et la phase aqueuse, ainsi l'utilisation d'un hydrogel obtenu.

La présente invention vise précisément à proposer un nouveau procédé de préparation d'un hydrogel injectable à base d'acide hyaluronique réticulé, ou l'un de ses sels, et éventuellement d'autres polymères biocompatibles et/ou tout autre ingrédient susceptible d'apporter un bénéfice au produit; procédé qui permet notamment :
- d'augmenter significativement l'élasticité et la rémanence de l'hydrogel obtenu, et donc sa performance clinique
- de diminuer la quantité de l'agent réticulant, molécule non dénuée d'une certaine toxicité, à introduire dans le mélange réactionnel au cours de la réticulation de l'acide hyaluronique, ceci afin d'améliorer le profil de sécurité de l'hydrogel injectable obtenu
- de limiter la présence de radicaux libres dérivés de l'oxygène au cours de la préparation du produit mais également au cours de son stockage (pendant sa durée de péremption) et lors de son utilisation chez le patient, ceci afin de réduire les effets délétères induits par ces entités indésirables sur l'acide hyaluronique mais également au niveau de la zone traitée chez le patient

Ainsi, la présente invention concerne, selon un premier de ses aspects, un procédé de préparation d'un hydrogel injectable à base d'acide hyaluronique réticulé, ou l'un de ses sels, et éventuellement d'autres polymères biocompatibles, comprenant au moins les étapes suivantes :
- préparation d'un hydrogel intégrant au moins une étape de réticulation de l'acide hyaluronique en solution aqueuse caractérisée en ce que la-dite étape de réticulation de l'acide hyaluronique est réalisée sous atmosphère inerte
- purification de l'hydrogel
- conditionnement de l'hydrogel en seringues ou en flacons
- stérilisation
en réalisant impérativement au moins une étape d'extraction de l'oxygène contenu dans l'hydrogel; étape d'extraction mise en oeuvre après l'étape de réticulation de l'acide hyaluronique sous atmosphère inerte et avant et/ou pendant l'étape de conditionnement de l'hydrogel; étape d'extraction consistant à réaliser, dans un récipient approprié, au moins un cycle d'extraction caractérisé par les phases successives suivantes :
- mise sous vide, à une pression p inférieure à la pression atmosphérique, du récipient contenant l'hydrogel pendant une durée t
- coupure du vide, au terme de la durée t, par l'introduction d'une quantité adaptée d'un gaz autre que l'oxygène

Selon un mode de réalisation de l'invention, l'atmosphère inerte utilisée est constituée d'un ou plusieurs gaz de grade médical.

Selon un mode de réalisation de l'invention, le ou les gaz (constitutifs de l'atmosphère inerte) utilisés pour substituer l'oxygène au cours de la réaction de réticulation sont choisis parmi l'hydrogène, l'hélium, l'azote et l'argon.

Selon un mode de réalisation de l'invention, la pression de l'atmosphère inerte est supérieure ou égale à la pression atmosphérique. Cette pression peut être fixe ou variable au cours du temps.

Selon l'invention, ce procédé de préparation permet également une extraction efficace de l'oxygène contenu dans l'hydrogel. Cette étape d'extraction est réalisée après l'étape de réticulation de l'acide hyaluronique et avant et/ou pendant l'étape de conditionnement de l'hydrogel. Il est à noter que, selon l'invention, la réticulation de l'acide hyaluronique est effectuée sous atmosphère inerte et qu'après la réticulation, une étape de purification (avantageusement par dialyse) ainsi que une ou plusieurs étapes d'homogénéisation sont à réaliser; étapes très généralement réalisées à l'air. Ainsi, il est important de préciser que ce n'est pas parce que l'étape de réticulation est effectuée sous atmosphère inerte qu'il n'y a pas réintroduction d'oxygène dans l'hydrogel à une étape ultérieure à la réticulation, d'où l'intérêt d'extraire cette oxygène efficacement avant ou pendant le conditionnement du gel.

La réalisation d'un ou plusieurs cycles d'extraction, impliquant la mise sous vide à une pression adaptée, suivie par la coupure de ce vide avec un gaz approprié ne renfermant pas d'oxygène (et donc pas d'air), permet de retirer la majeure partie de l'oxygène contenu dans le produit. En effet, lors de la mise sous vide, réalisée avantageusement en dynamique, les molécules de gaz contenues dans l'hydrogel, et donc notamment les molécules d'oxygène, sont emportées par le vide appliqué. Le fait de casser ce vide avec un gaz ne contenant pas d'oxygène permet de ne pas réintégrer de l'oxygène dans le produit.

La réalisation de plusieurs cycles d'extraction permet ainsi de retirer une quantité toujours plus importante d'oxygène, en cumulé, au fil de ces cycles d'extraction.

Il est à noter que le déroulement de l'opération d'extraction de l'oxygène, réalisée avantageusement dans un récipient de type chambre à vide, peut être observé visuellement si le récipient contenant l'hydrogel est transparent. Lors de la mise sous vide du produit, on constate une expansion de l'hydrogel. On entend par expansion de l'hydrogel le fait que celui-ci prend un volume plus important au sein du récipient. Lorsque le vide est cassé, l'hydrogel reprend immédiatement un volume proche de celui qu'il avait initialement, ce qui participe au fait de chasser l'oxygène résiduel contenu dans le gel. En multipliant les cycles d'extraction, on constate que l'expansion de l'hydrogel dans le récipient devient de moins en moins importante au fil des cycles, ce qui est le signe qu'il y a de moins en moins de gaz et donc d'oxygène dans le gel.

Pour casser le vide, un gaz léger et de petite taille comme l'hélium est avantageusement choisi pour faciliter la procédure d'extraction de l'oxygène.

D'autre part, compte tenu de la nature injectable du produit, un gaz de grade médical est avantageusement sélectionné afin d'assurer une totale sécurité du produit fabriqué.

Il est important de préciser que l'étape d'extraction de l'oxygène du procédé de préparation selon l'invention apporte des avantages conséquents à son fabricant par rapport à la technique dite de « dégazage » de l'art antérieur. En effet, le dégazage, lui, est destiné à éliminer des petites bulles d'air présentes dans un gel, ce qui permet d'avoir un aspect visuel de celui-ci davantage apprécié des utilisateurs finaux (les praticiens et les patients), et non à retirer une majeure partie de l'oxygène contenu dans le produit. Par rapport à cette technique de dégazage de l'art antérieur, la solution proposée permet :
- elle aussi de retirer les bulles d'air présentes dans le gel afin d'en améliorer l'aspect visuel
- d'extraire efficacement l'oxygène contenu dans le gel, au fil des cycles d'extraction
- un gain de temps conséquent car les cycles d'extraction de l'oxygène à appliquer peuvent être de très courtes durées, contrairement à un dégazage de l'art antérieur qui implique d'attendre que l'air s'échappe petit à petit du gel au cours de sa mise sous vide (ceci pouvant induire, en plus du temps important nécessaire à sa réalisation, une déshydratation de l'hydrogel)

Il est également important de préciser que l'homme de l'art, en fonction des caractéristiques spécifiques de sa formulation (concentration en acide hyaluronique, taux de réticulation, présence d'autres composés, ...), saura sélectionner les conditions appropriées pour extraire l'oxygène de son produit et les optimiser (pression p et durée t pour la mise sous vide, quantité de gaz pour casser le vide, nombre de cycles d'extraction à effectuer, ....).

Selon un mode de réalisation de l'invention, une seule étape d'extraction de l'oxygène contenu dans l'hydrogel est réalisée au cours de la fabrication du produit injectable.

Selon un autre mode de réalisation de l'invention, entre 2 et 4 étapes d'extraction de l'oxygène contenu dans l'hydrogel sont réalisées au cours de la fabrication du produit injectable.

Selon un mode de réalisation de l'invention, une étape d'extraction de l'oxygène contenu dans l'hydrogel est réalisée avant ou pendant le conditionnement de cet hydrogel dans des seringues ou dans des flacons.

Selon un mode de réalisation de l'invention, une étape d'extraction de l'oxygène contenu dans l'hydrogel est réalisée avant l'ajout dans le gel d'un ingrédient, par exemple un principe actif, susceptible d'être oxydé par l'oxygène contenu dans ce gel.

Selon un mode particulier de réalisation de l'invention, une étape d'extraction de l'oxygène (réalisée comme décrite précédemment) contenu dans le mélange réactionnel est également effectuée avant l'étape de réticulation de l'acide hyaluronique sous atmosphère inerte. Cette étape additionnelle permet de retirer l'oxygène éventuellement présent dans le mélange réactionnel avant réticulation, ceci en particulier si les étapes antérieures à la réticulation (par exemple, la préparation de la solution aqueuse d'acide hyaluronique) ont été réalisées à l'air, c'est-à-dire en l'absence d'atmosphère inerte.

Selon un mode de réalisation de l'invention, l'étape d'extraction de l'oxygène est effectuée en appliquant entre 1 et 50 cycles d'extraction caractérisés par la mise sous vide et par la coupure de ce vide à l'aide d'un gaz autre que l'oxygène. Avantageusement, le nombre de cycles appliqués est compris entre 2 et 10, de préférence entre 3 et 6, au cours de l'étape d'extraction de l'oxygène.

Selon un mode de réalisation de l'invention, la stérilisation du produit est effectuée à la chaleur, préférentiellement à la chaleur humide.

Selon un autre mode de réalisation de l'invention, la stérilisation est effectuée selon un procédé aseptique.

Selon un mode de réalisation préféré de l'invention, l'hydrogel est conditionné dans des seringues.

Selon un mode de réalisation de l'invention, le gaz utilisé pour casser le vide lors de l'extraction de l'oxygène est un gaz plus léger que l'air.

Selon un mode de réalisation de l'invention, le gaz utilisé est un gaz inerte.

Selon un mode de réalisation de l'invention, le gaz utilisé est un gaz de grade médical.

Selon un mode de réalisation de l'invention, le gaz utilisé est choisi parmi l'hydrogène, l'hélium, l'azote et l'argon. Avantageusement, le gaz utilisé est l'azote ou l'hélium.

Selon un mode de réalisation de l'invention, le gaz utilisé est un mélange de différents gaz autres que l'oxygène.

Selon un mode de réalisation préféré de l'invention, la mise sous vide de l'hydrogel est effectuée en dynamique (le système créant le vide, avantageusement une pompe à vide, est connecté en permanence au récipient contenant l'hydrogel afin d'en maintenir la pression souhaitée) plutôt qu'en statique (le récipient contenant l'hydrogel est isolé du système créant le vide une fois que la pression a atteint la valeur souhaitée).

Selon un mode de réalisation préféré de l'invention, la pression p appliquée au cours de la mise sous vide est inférieure à 100 mbar, avantageusement inférieure à 50 mbar.

Selon l'invention, l'étape d'extraction de l'oxygène est effectuée dans un récipient approprié. Ce récipient peut par exemple être une chambre à vide ou un dessiccateur possédant une vanne qui permet de rendre la chambre hermétique ou de casser le vide. La mise sous vide est avantageusement assurée par l'utilisation d'une pompe à vide.

Selon un mode de réalisation de l'invention, la durée t de mise sous vide pour un cycle d'extraction est comprise entre 1 minute et 60 minutes, préférentiellement entre 5 minutes et 25 minutes.

On entend par étape de réticulation de l'acide hyaluronique l'étape permettant de ponter les chaînes d'acide hyaluronique les unes aux autres par des liaisons covalentes. De manière générale, l'étape de réticulation de l'acide hyaluronique commence lorsque le réticulant est mis en contact avec l'acide hyaluronique puis homogénéisé au sein du mélange réactionnel et elle se termine lorsque l'homme de l'art considère que la cinétique de réaction de pontage des chaînes d'acide hyaluronique par le réticulant a atteint un niveau négligeable.

Dans le cas de la présente invention, comme décrit précédemment, l'étape de réticulation est impérativement réalisée sous atmosphère inerte.

La préparation de l'hydrogel à base d'acide hyaluronique réticulé est effectuée préférentiellement selon les méthodes décrites dans l'art antérieur.

On pourra par exemple citer les demandes WO 97/04012, WO 2004/09222 et WO 2009/071697 pour la fabrication d'un hydrogel à base d'acide hyaluronique réticulé.

Un procédé avantageux selon l'invention pour la fabrication d'un gel à base d'acide hyaluronique réticulé comprend au moins les étapes suivantes :
- préparation d'une solution aqueuse d'acide hyaluronique sous atmosphère inerte
- réticulation de l'acide hyaluronique sous atmosphère inerte
- neutralisation du gel obtenu à pH environ égal à 7.0 et élimination du réticulant par purification, par exemple par dialyse dans une solution physiologique
- extraction optionnelle de l'oxygène selon la méthode décrite précédemment
- ajout optionnel de molécules complémentaires comme par exemple des vitamines et/ou un principe actif
- homogénéisation du gel
- extraction de l'oxygène selon la méthode décrite précédemment
- conditionnement en flacons ou seringues
- stérilisation

Un autre procédé avantageux selon l'invention pour la fabrication d'un gel à base d'acide hyaluronique réticulé comprend au moins les étapes suivantes :
- préparation d'une solution aqueuse d'acide hyaluronique
- extraction de l'oxygène selon la méthode décrite précédemment
- réticulation de l'acide hyaluronique sous atmosphère inerte
- neutralisation du gel obtenu à pH environ égal à 7.0 et élimination du réticulant par purification, par exemple par dialyse dans une solution physiologique
- extraction optionnelle de l'oxygène selon la méthode décrite précédemment
- ajout optionnel de molécules complémentaires comme par exemple des vitamines et/ou un principe actif
- homogénéisation du gel
- extraction de l'oxygène selon la méthode décrite précédemment
- conditionnement en flacons ou seringues
- stérilisation

Le procédé selon l'invention permet de réaliser au moins impérativement l'étape de réticulation sous atmosphère inerte mais également d'extraire de manière extrêmement efficace l'oxygène contenu dans l'hydrogel avant et/ou pendant le conditionnement de celui-ci en flacons ou en seringues.

De manière tout à fait surprenante et très avantageuse, comme illustré dans les exemples, l'application du procédé selon l'invention permet de significativement améliorer les propriétés de l'hydrogel obtenu selon ce procédé. On constate alors :
- une amélioration significative des propriétés mécaniques/rhéologiques de l'hydrogel et notamment de son élasticité (augmentation du module élastique G')
- la possibilité de réduire la quantité de réticulant utilisée (molécule possédant un certain niveau de toxicité) pour réticuler l'acide hyaluronique, dans le but d'améliorer le niveau de sécurité de l'hydrogel injectable obtenu
- une amélioration significative de la résistance de l'hydrogel à la dégradation
- une meilleure stabilité de l'hydrogel au cours du temps

Il est important de préciser que le procédé selon l'invention a un impact direct fort sur la structure de l'hydrogel au cours de l'étape cruciale de réticulation mais également au cours d'autres étapes comme celle de stérilisation. Le procédé selon l'invention confère alors un bénéfice considérable au produit issu de l'invention. Il permet d'une part d'obtenir un produit plus performant mais également un produit plus sûr, ce qui est absolument majeur pour viser une tolérance encore plus grande des nombreux traitements utilisant de l'acide hyaluronique réticulé.

Ainsi, on observe que le produit issu de l'invention a une meilleure capacité à créer du volume (du fait de sa plus forte élasticité) et ceci sur le plus long terme (du fait de sa plus forte capacité à résister à la dégradation mais également du fait de sa meilleure stabilité), d'où une meilleure efficacité clinique dans les domaines de l'esthétique ou de la médecine. Le procédé selon l'invention donne également la possibilité de réduire la quantité de réticulant utilisée (agent chimique non dénué d'une certaine toxicité), pour un même taux de réticulation effectif visé, ce qui permet alors d'améliorer le profil de sécurité du produit injectable obtenu.

La présente invention concerne, selon un deuxième de ses aspects, un hydrogel injectabte à base d'acide hyaluronique réticulé obtenu selon le procédé de préparation décrit ci-dessus. Cet hydrogel peut également contenir d'autres polymères biocompatibles et tout autre ingrédient susceptible d'apporter un bénéfice au produit injectable. Cet hydrogel se caractérise par des propriétés mécaniques/rhéologiques et de rémanence améliorées par rapport à un produit de l'art antérieur qui n'a pas subi le procédé de préparation selon l'invention. En effet, l'hydrogel selon l'invention possède une plus forte élasticité et une plus grande résistance à la dégradation (par les facteurs de dégradation *in vivo* comme la dégradation par hydrolyse thermique, la dégradation radicalaire et la dégradation enzymatique), pour viser une meilleure performance clinique du produit injecté.

L'hydrogel injectable selon l'invention est également réticulé plus efficacement, ce qui autorise alors d'utiliser moins d'agent réticulant (molécule possédant un certain niveau de toxicité) pour ponter les chaînes d'acide hyaluronique de manière équivalente à un gel de l'art antérieur, renforçant ainsi le profil de sécurité du produit obtenu. Cette caractéristique essentielle du produit selon l'invention permet de viser une meilleure tolérance des traitements injectables à base d'acide hyaluronique réticulé, par exemple en diminuant les effets secondaires sur le court et/ou sur le long terme. Il est aussi important de noter que la meilleure maîtrise/efficacité de la réticulation peut permettre de limiter l'apparition dans le mélange réactionnel de petites masses moléculaires d'acide hyaluronique (en particulier, sous conditions basiques ou acides et à plus haute température comme c'est le cas lors de la réticulation); entités décrites dans la littérature comme étant pro-inflammatoires et donc génératrices d'effets indésirables.

L'hydrogel injectable selon l'invention est également plus stable au cours de son stockage, c'est-à-dire au cours de sa durée de péremption. Ainsi, cela permet une meilleure conservation des propriétés mécaniques/rhéologiques du gel (le module élastique G' est par exemple moins altéré au cours du temps - on observe ainsi une diminution moins forte de ce paramètre au cours du temps), mais cela permet également de limiter l'apparition de molécules d'acide hyaluronique de faibles masses moléculaires (décrites précédemment) au cours du temps. Cela peut ainsi permettre au fabricant de prolonger la durée de péremption de son produit. Sans vouloir se lier à une explication, cet avantage est probablement dû au fait que le procédé selon l'invention permet de fortement limiter la quantité d'oxygène au sein de l'hydrogel (les radicaux libres de l'oxygène ayant un effet délétère sur l'acide hyaluronique en coupant ses chaînes) mais aussi au fait que le procédé selon l'invention permet de préparer un produit avec une structure plus « robuste » (comme démontré par le fait qu'il possède une meilleure élasticité et une meilleure résistance à la dégradation).

D'autre part, l'extraction de l'oxygène selon l'invention, grâce à son efficacité, permet de renforcer la sécurité du patient par rapport aux produits de l'art antérieur. En effet, l'utilisation de l'hydrogel selon l'invention permet de limiter fortement l'apport d'oxygène dans les tissus (induit par l'injection de l'hydrogel contenant de l'oxygène), ce qui participe alors à réduire la création de radicaux libres au niveau de la zone injectée (stress oxydatif de l'organisme), apportant ainsi un bénéfice vis-à-vis du gel (moins forte dégradation de celui-ci) mais également vis-à-vis des tissus environnants qui sont alors moins agressés par les radicaux libres (ce qui participe alors à limiter l'action inflammatoire accompagnant systématiquement tout type d'injection).

Selon l'invention, l'hydrogel contient de l'acide hyaluronique ou l'un de ses sels, et en particulier ses sels acceptables d'un point de vue physiologique comme les sels de sodium, calcium, zinc, potassium, avantageusement le sel de sodium. L'acide hyaluronique peut être d'origine animale ou obtenu par fermentation bactérienne. Il peut avoir une masse moléculaire de quelques daltons à plusieurs millions de daltons, avantageusement d'environ 0.1 à 4 millions de daltons.

Selon un aspect de l'invention, l'hydrogel peut être à base d'un dérivé de l'acide hyaluronique, c'est-à-dire à base d'une molécule obtenue en modifiant par voie chimique, ou par toute autre voie, la molécule d'acide hyaluronique.

Selon un aspect de l'invention, la concentration totale de l'hydrogel en acide hyaluronique, ou l'un de ses sels, est comprise entre 0.01 et 50 mg/ml, de préférence entre 1 et 35 mg/ml, avantageusement entre 8 et 30 mg/ml.

Selon un aspect de l'invention, l'hydrogel est réticulé préférentiellement selon les procédés décrits dans l'art antérieur.

Le ou les agents réticulants qui interviennent dans la réticulation peuvent être identiques ou différents. Ce sont généralement des réticulants bi- ou poly-fonctionnels de différents types et ils peuvent par exemple être sélectionnés parmi la divinylsulfone, les époxy bi- ou poly-fonctionnels, les carbodiimides et le formaldéhyde. On choisit de préférence les agents de la famille des époxy bi- ou poly-fonctionnels et notamment le 1,4-butanedioldiglycidyléther (BDDE), le diépoxy-octane ou le 1,2-bis-(2,3-époxypropyl)-2,3-éthylène. On préfère tout particulièrement utiliser le BDDE.

Les températures de réticulation sont généralement comprises entre environ 15°C et 60°C.

Les durées de réticulation sont généralement de plusieurs heures, avantageusement de plus de 1h jusqu'à environ 48h.

Selon un aspect de l'invention, l'hydrogel selon l'invention contient une ou plusieurs substances actives d'origine naturelle ou synthétique à action pharmacologique ou non, comme par exemple les antioxydants, les anti-inflammatoires, les antiseptiques, les antibactériens, les antifongiques, les anticancéreux, les anesthésiques locaux, les protéines, les hormones, seuls ou en combinaison. Ces substances actives sont soit dispersées dans l'hydrogel, soit greffées à un ou plusieurs des polymères de l'hydrogel, soit contenues/encapsulées dans un autre matériau lui-même dispersé au sein de l'hydrogel. Dans ce dernier cas, à titre d'exemple, on pourra citer l'encapsulation d'une substance active, comme un anti-inflammatoire, dans des microsphères à base d'un polymère dérivé de l'acide polylactique ou du poly-ε-caproladone.

Selon un aspect de l'invention, l'hydrogel selon l'invention contient de la lidocaïne dispersée au sein de sa matrice réticulée.

Selon un aspect de l'invention, l'hydrogel selon l'invention contient un ou plusieurs composés d'origine biologique comme des cellules, des plaquettes enrichies, des gènes, des fragments d'ADN ou des facteurs de croissance. Ces composés sont préférentiellement dispersés dans l'hydrogel, mais ils peuvent également être greffés à un ou plusieurs des polymères de l'hydrogel ou contenus/encapsulés dans un autre matériau lui-même dispersé au sein de l'hydrogel.

Selon un aspect de l'invention, l'hydrogel selon l'invention contient des polymères qui sont dispersés au sein de la matrice réticulée de l'hydrogel. On peut citer par exemple les polymères de la famille des polysaccharides, les polyesters, les polyanhydrides, les polyphosphazenes, les poly-ε-capralactones, les acides polylactiques et leurs dérivés, les acides polyvinyliques, les polyacrylamides, la N-vinylpyrrolidone et les polymères acryliques et dérivés biologiquement acceptables.

Selon un aspect de l'invention, l'hydrogel selon l'invention contient des substances minérales qui sont dispersées au sein de la matrice réticulée de l'hydrogel. On peut citer par exemple l'hydroxyapatite ou les phosphates tricalciques comme le β tricalcium phosphate.

La présente invention concerne, selon un troisième de ses aspects, l'utilisation chez l'Homme ou chez l'animal de l'hydrogel injectable obtenu selon ledit procédé de préparation dans les domaines de l'esthétique et de la médecine.

L'hydrogel injectable selon l'invention est notamment utilisé pour :
- combler des volumes
- générer des espaces au sein de certains tissus, favorisant ainsi leur fonctionnement optimal
- remplacer des liquides physiologiques ou des tissus déficients
- stimuler ou favoriser la régénération des tissus
- hydrater et protéger des tissus
- délivrer des substances susceptibles d'apporter un bénéfice à l'organisme et notamment des substances actives et/ou des biologiques

A titre d'exemple, on peut citer les utilisations de l'hydrogel dans les cas suivants :
- la formulation d'une composition injectable en intradermique ou en sous-cutané pour l'amélioration de la qualité de la peau ou le comblement des rides ou la restauration des volumes du visage (pommettes, menton, lèvres, nez, ...) ou du corps
- la formulation d'une composition injectable à usage dentaire par exemple pour combler des poches parodontales et/ou pour stimuler la régénération des tissus autour de la dent
- la formulation d'une composition injectable en intra-oculaire, notamment pour des applications au cours de la chirurgie de la cataracte, du glaucome, de la presbytie ou du vitré
- la formulation de compositions injectables en intra-articulaire pour des applications en orthopédie ou en rhumatologie, notamment dans le cadre de la viscosupplémentation du liquide synovial déficient pour le traitement de l'arthrose mais également de la reconstruction osseuse ou de la régénération du cartilage
- la formulation d'une composition injectable en urologie pour des applications dans le traitement de l'incontinence urinaire ou fécale
- la formulation d'une composition injectable utilisée en médecine ou chirurgie générale dans le cadre du traitement de la fibrose ou pour améliorer la cicatrisation des plaies
- la formulation d'une composition pharmaceutique injectable permettant la libération retardée et/ou contrôlée de substances actives et/ou de biologiques pour différentes applications médicales

### EXEMPLES

L'invention va maintenant être illustrée, de manière non limitative, par les exemples suivants.

Le hyaluronate de sodium (NaHA) et l'ensemble des autres composés utilisés dans les exemples possèdent un haut niveau de pureté.

Les propriétés rhéologiques (mesure du module élastique G') des gels sont mesurées à 25°C à l'aide d'un rhéomètre à contrainte imposée (TA AR2000) et d'une géométrie cône/plan 4 cm-2°.

### Exemple 1 : Préparation d'un gel à base de NaHA réticulé selon l'invention

Dans un mélangeur de laboratoire sous atmosphère inerte composée de 100% de gaz hélium à la pression de 1.4 bar, à température ambiante, on introduit 24 g d'une solution aqueuse de NaOH à 0.25 N puis 2.5 g d'une poudre de hyaluronate de sodium (NaHA) de masse moléculaire environ égale à 1.8 MDa et possédant un taux d'humidité de 7.8%. L'hydratation de la poudre dure 1h30, avec une homogénéisation mécanique régulière. 1.3 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes avant augmentation de la température à 50°C. Après 2h45 à la température de 50°C, le réticulat obtenu est introduit dans un bécher contenant une solution de tampon phosphate et du HCl afin d'obtenir un pH=7.3 et une concentration en acide hyaluronique égale à 25 mg/ml. On laisse le gel gonfler pendant 24h à température ambiante dans cette solution et on le purifie ensuite par dialyse pendant environ 24h en utilisant des membranes de dialyse à base de cellulose (seuil de rétention = 10 000 Da) dans une solution de tampon phosphate jusqu'à obtenir une concentration en acide hyaluronique de 20 mg/ml.

Après homogénéisation de 15 minutes à la spatule, on réalise ensuite une extraction de l'oxygène contenu dans le gel en le plaçant dans une chambre à vide, connectée à une pompe à vide, et en effectuant 6 cycles d'extraction consécutifs caractérisés par les étapes suivantes :
- la chambre à vide est mise sous vide dynamique à une pression de 11 mbar pendant 15 minutes pour les premier, deuxième et troisième cycles d'extraction et 5 minutes pour les 3 cycles suivants
- le vide est coupé à la fin de chaque cycle en introduisant une quantité adaptée d'hélium dans la chambre à vide

Le gel est ensuite conditionné dans des seringues en verre de 1 ml qui sont stérilisées à l'autoclave à 121°C pendant 20 minutes. Soit G1S l'hydrogel stérilisé ainsi obtenu.

On réalise exactement la même préparation que précédemment sauf que :
- l'on utilise une atmosphère inerte composée de 100% de gaz azote à la pression de 1.4 bar; l'azote remplaçant l'hélium
- l'on utilise 1.0 g d'une solution de 1,4-butanedioldiglycidyléther (BDDE) dilué au 1/5^{ème} dans la soude 0.25 N (contre 1.3 g pour le gel G1S)

Soit G2S le gel ainsi obtenu.

### Exemple 2 : Préparation d'un gel à base de NaHA réticulé selon l'art antérieur (comparatif)

Dans un mélangeur de laboratoire (air et pression atmosphérique), à température ambiante, on introduit 24 g d'une solution aqueuse de NaOH à 0.25 N puis 2.5 g d'une poudre de hyaluronate de sodium (NaHA) de masse moléculaire environ égale à 1.8 MDa et possédant un taux d'humidité de 7.8%. L'hydratation de la poudre dure 1h30, avec une homogénéisation mécanique régulière. 1.3 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes avant augmentation de la température à 50°C. Après 2h45 à la température de 50°C, le réticulat obtenu est introduit dans un bécher contenant une solution de tampon phosphate et du HCl afin d'obtenir un pH=7.3 et une concentration en acide hyaluronique égale à 25 mg/ml. On laisse le gel gonfler pendant 24h puis on le purifie par dialyse pendant environ 24h en utilisant des membranes de dialyse à base de cellulose (seuil de rétention = 10 000 Da) dans une solution de tampon phosphate jusqu'à obtenir une concentration en acide hyaluronique de 20 mg/ml.

Après homogénéisation de 15 minutes à la spatule, le gel est ensuite conditionné dans des seringues en verre de 1 ml qui sont stérilisées à l'autoclave à 121°C pendant 20 minutes. Soit G3S l'hydrogel stérilisé ainsi obtenu.

### Exemple 3 : Caractérisation des gels G1S, G2S et G3S par rhéologie (comparatif)

On mesure les modules élastiques G' à 0.7 Hz des hydrogels **G1S,** G2S et G3S.

| **Gel testé** | **G' (0.7 Hz) en Pa** (moyenne sur 3 valeurs mesurées par gel) |
|---|---|
| Gel G1S (selon l'invention) | 430 |
| Gel G2S (selon l'invention) | 342 |
| Gel G3S (selon l'art antérieur) | 333 |

On constate que le gel G1S selon l'invention, préparé exactement avec la même quantité de réticulant que le gel G3S, possède une élasticité significativement plus élevée que ce dernier. Cela confère au gel G1S un bénéfice considérable car permettant d'augmenter la capacité du gel à pousser les tissus et donc à créer du volume *in vivo* (point clé pour l'efficacité clinique de nombreuses formulations à base d'acide hyaluronique réticulé en esthétique (cas par exemple des produits dédiés à la restauration des volumes du visage) et en médecine (cas par exemple des produits dédiés au traitement de l'incontinence)).

On constate également que le gel G2S selon l'invention, préparé lui avec une quantité de réticulant (BDDE) significativement inférieure à celle de G3S, possède une élasticité similaire/équivalente à ce dernier.

Ainsi, de manière tout à fait surprenante, le procédé selon l'invention permet :
- d'améliorer les propriétés biomécaniques/rhéologiques du gel et donc d'améliorer la performance clinique du produit utilisé
- de diminuer la quantité de réticulant (agent chimique non dénué d'une certaine toxicité) introduit pour ponter les chaînes d'acide hyaluronique et donc d'améliorer le profil de sécurité du produit utilisé

### Exemple 4 : Caractérisation des gels G1S, G2S et G3S - Résistance à la dégradation (comparatif)

Dans 3 pots transparents de 30 ml, on introduit respectivement 5 ml des gels G1S, G2S et G3S. On ajoute ensuite 0.5 ml d'une solution de peroxyde d'hydrogène (Ph. Eur.) dans chacun des 3 gels et on mélange à la spatule pendant 5 minutes. On ferme ensuite les 3 pots et on observe l'écoulement du gel au cours du temps, à température ambiante, par retournement régulier de chacun des 3 pots en simultané.

A t=0, on constate que les 3 gels s'écoulent peu et de la même manière.

Au bout de 6h à température ambiante, on constate que les gels G2S (selon l'invention) et G3S s'écoulent de la même manière et significativement plus que le gel G1S selon l'invention.

Au bout de 12h à température ambiante, on constate que les gels G2S (selon l'invention) et G3S sont extrêmement fluides (s'écoulent immédiatement dès qu'on les retourne) alors que le gel G1S a encore une consistance de produit visqueux (bien que beaucoup plus fluide qu'à t=0).

On déduit de cette expérience que :
- le gel G1S selon l'invention, bien que préparé exactement avec la même quantité de réticulant que le gel G3S, possède une viscosité supérieure au cours du temps par rapport à ce dernier. Les gels G1S et G3S sont tous les deux dégradés au cours du temps par la solution de peroxyde d'hydrogène mais le gel G1S possède une meilleure résistance à la dégradation que G3S.
- Les gels G2S et G3S possèdent des résistances similaires à la dégradation par la solution de peroxyde d'hydrogène mais il est important de préciser que le produit G2S selon l'invention a été préparé avec une quantité de réticulant significativement inférieure à G3S.

Ainsi, de manière tout à fait surprenante, le procédé selon l'invention permet d'améliorer la résistance à la dégradation d'un gel à base d'acide hyaluronique réticulé. Cela confère un avantage clé au produit développé dans le domaine de l'esthétique ou de la médecine car la formulation va ainsi pouvoir agir sur une plus longue période (amélioration de la rémanence) au niveau de la zone injectée/traitée.

### Exemple 5 : Caractérisation des gels G1S, G2S et G3S - Stabilité au cours du temps (comparatif)

On mesure les modules élastiques G' à 0.7 Hz des hydrogels G1S, G2S et G3S après 12 mois de stockage à température ambiante et on compare les valeurs obtenues à celles mesurées à t=0.

| **Gel testé** | **Variation du G' (0.7 Hz)** |
|---|---|
| Gel G1S (selon l'invention) | -9% |
| Gel G2S (selon l'invention) | -10% |
| Gel G3S (selon l'art antérieur) | -26% |

Après 12 mois à température ambiante, on constate que les modules élastiques G' des gels G1S et G2S selon l'invention ont significativement moins diminués que celui du gel G3S. La stabilité des gels selon l'invention est donc améliorée par rapport à celle du gel de l'art antérieur.

### Exemple 6 : Préparation d'un gel à base de NaHA réticulé selon l'invention et d'un gel selon l'art antérieur (comparatif)

Dans un mélangeur de laboratoire (air et pression atmosphérique), à température ambiante, on introduit 19 g d'une solution aqueuse de NaOH à 0.25 N puis 2.1 g d'une poudre de hyaluronate de sodium (NaHA) de masse moléculaire environ égale à 1.5 MDa et possédant un taux d'humidité de 6.1%. L'hydratation de la poudre dure 1h30, avec une homogénéisation mécanique régulière. 0.8 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes.

Toujours dans le mélangeur de laboratoire, on réalise ensuite une étape d'extraction de l'oxygène (durée totale de l'étape = 8 minutes) contenu dans le mélange réactionnel en effectuant 1 cycle d'extraction caractérisé de la manière suivante :
- l'enceinte du mélangeur est mise sous vide dynamique à une pression de 52 mbar pendant 6 minutes
- le vide est coupé en introduisant une quantité adaptée d'azote dans l'enceinte du mélangeur

La température au sein de l'enceinte du mélangeur est ensuite montée à 50°C pendant 3h00 pour réticuler le mélange réactionnel sous atmosphère inerte composée de 100% de gaz azote à la pression de 1.9 bar. A la fin de cette période, le réticulat obtenu est introduit dans un bécher contenant une solution de tampon phosphate et du HCl afin d'obtenir un pH=7.3 et une concentration en acide hyaluronique égale à 25 mg/ml. On laisse le gel gonfler pendant 24h à température ambiante dans cette solution et on le purifie ensuite par dialyse pendant environ 24h en utilisant des membranes de dialyse à base de cellulose (seuil de rétention = 10 000 Da) dans une solution de tampon phosphate jusqu'à obtenir une concentration en acide hyaluronique de 20 mg/ml.

Après homogénéisation de 15 minutes à la spatule, on réalise ensuite une extraction de l'oxygène contenu dans le gel en le plaçant dans une chambre à vide, connectée à une pompe à vide, et en effectuant 4 cycles d'extraction consécutifs caractérisés par les étapes suivantes :
- la chambre à vide est mise sous vide dynamique à une pression de 20 mbar pendant 10 minutes pour les premier, deuxième et troisième cycles d'extraction et 5 minutes pour le quatrième cycle
- le vide est coupé à la fin de chaque cycle en introduisant une quantité adaptée d'azote dans la chambre à vide

Le gel est ensuite conditionné dans des seringues en verre de 1 ml qui sont stérilisées à l'autoclave à 121°C pendant 20 minutes. Soit G4S l'hydrogel stérilisé (selon l'invention) ainsi obtenu.

On réalise exactement la même préparation que précédemment sauf que :
- 0.9 g d'une solution de 1,4-butanedioldiglycidyléther (BDDE) dilué au 1/5^{ème} dans la soude 0.25 N sont ajoutés au milieu réactionnel (au lieu de 0.8 g pour le gel G4S)
- un temps de repos de 8 minutes après ajout du BDDE et homogénéisation mécanique de 15 minutes est effectué à la place de l'étape d'extraction de l'oxygène contenu dans le mélange réactionnel
- la réticulation est effectuée à l'air (c'est-à-dire pas sous atmosphère inerte)
- une étape d'extraction de l'oxygène n'est pas réalisée après purification du gel par dialyse et avant conditionnement en seringues

Soit G5S le gel stérilisé (selon l'art antérieur) ainsi obtenu.

On mesure les modules élastiques G' à 0.7 Hz des hydrogels G4S et G5S.

| **Gel testé** | **G' (0.7 Hz) en Pa** |
|---|---|
| | (moyenne sur 3 valeurs mesurées par gel) |
| Gel G4S | 393 |
| (selon l'invention) | |
| Gel G5S | 317 |
| (selon l'art antérieur) | |

Comme démontré dans l'exemple 3, on confirme dans ce nouvel exemple que le procédé de préparation selon l'invention a une action forte sur la structure d'un hydrogel à base d'acide hyaluronique réticulé.

En effet, bien qu'ayant utilisé une plus forte quantité d'agent réticulant (plus de 10% de BDDE en plus) pour la préparation du gel G5S selon l'art antérieur, on constate que ce produit est moins réticulé et moins robuste (la valeur du G' à 0.7 Hz est inférieure à celle du gel G4S) que le gel G4S selon l'invention.

Ainsi, cet exemple permet de confirmer que, de manière tout à fait surprenante, le procédé selon l'invention permet :
- d'améliorer les propriétés biomécaniques/rhéologiques du gel et donc d'améliorer la performance clinique du produit utilisé
- de diminuer la quantité de réticulant (agent chimique non dénué d'une certaine toxicité) introduit dans le mélange réactionnel pour réticuler les chaînes d'acide hyaluronique et donc améliorer le profil de sécurité du produit utilisé

## Revendications

1. Procédé de préparation d'un hydrogel injectable à base d'acide hyaluronique réticulé, ou l'un de ses sels, et éventuellement d'autres polymères biocompatibles, comprenant au moins les étapes suivantes :
- préparation d'un hydrogel intégrant au moins une étape de réticulation de l'acide hyaluronique en solution aqueuse **caractérisée en ce que** la-dite étape de réticulation de l'acide hyaluronique est réalisée sous atmosphère inerte ;
- purification de l'hydrogel ;
- conditionnement de l'hydrogel en seringues ou en flacons ;
- stérilisation ;
et en réalisant impérativement au moins une étape d'extraction de l'oxygène contenu dans l'hydrogel; la-dite étape d'extraction étant mise en oeuvre après l'étape de réticulation de l'acide hyaluronique sous atmosphère inerte et avant et/ou pendant l'étape de conditionnement de l'hydrogel; la-dite étape d'extraction consistant à réaliser, dans un récipient approprié, au moins un cycle d'extraction **caractérisé par** les phases successives suivantes :
- mise sous vide, à une pression p inférieure à la pression atmosphérique, du récipient contenant l'hydrogel pendant une durée t;
- coupure du vide, au terme de la durée t, par l'introduction d'une quantité adaptée d'un gaz autre que l'oxygène.

2. Procédé selon la revendication 1, **caractérisé en ce que**, dans le cas de l'étape d'extraction de l'oxygène, le gaz autre que l'oxygène est un gaz inerte ou un mélange de gaz inertes, préférentiellement plus légers que l'air.

3. Procédé selon l'une des revendications 1 à 2, **caractérisé en ce que** l'atmosphère inerte est constituée par l'un ou plusieurs des gaz suivants : hydrogène, hélium, azote et argon.

4. Procédé selon la revendication 1, **caractérisé en ce que** l'atmosphère inerte est à une pression supérieure ou égale à la pression atmosphérique.

5. Procédé selon l'une des revendications 1 à 4, **caractérisé en ce que**, dans le cas de l'étape d'extraction de l'oxygène, la mise sous vide est effectuée à une pression inférieure à 100 mbar, préférentiellement inférieure à 50 mbar.

6. Procédé selon l'une des revendications 1 à 5, **caractérisé en ce que**, dans le cas de l'étape d'extraction de l'oxygène, la mise sous vide est effectuée en dynamique.

7. Procédé selon l'une des revendications 1 à 6, **caractérisé en ce que**, dans le cas de l'étape d'extraction de l'oxygène, la durée de mise sous vide est comprise entre 1 minute et 60 minutes, préférentiellement entre 5 minutes et 25 minutes.

8. Procédé selon l'une des revendications 1 à 7, **caractérisé en ce que**, dans le cas de l'étape d'extraction de l'oxygène, le nombre d'étapes d'extraction de l'oxygène contenu dans l'hydrogel injectable au cours de sa fabrication est compris entre 1 et 4.

9. Procédé selon l'une des revendications 1 à 8, **caractérisé en ce que**, dans le cas de l'étape d'extraction de l'oxygène, chaque étape d'extraction de l'oxygène réalisée au cours de la fabrication de l'hydrogel injectable comprend un nombre de cycles d'extraction compris entre 1 et 50, préférentiellement entre 2 et 10, encore préférentiellement entre 3 et 6.

10. Procédé selon l'une des revendications 1 à 9, **caractérisé en ce que**, avant l'étape de réticulation de l'acide hyaluronique sous atmosphère inerte, une étape d'extraction de l'oxygène contenu dans le mélange réactionnel est effectuée.

11. Procédé de préparation d'un hydrogel injectable à base d'acide hyaluronique réticulé, selon l'une des revendications 1 à 10, **caractérisé en ce qu'**il comprend au moins les étapes suivantes :
- préparation d'une solution aqueuse d'acide hyaluronique sous atmosphère inerte
- réticulation de l'acide hyaluronique sous atmosphère inerte
- élimination du réticulant par purification
- extraction optionnelle de l'oxygène
- ajout optionnel de molécules complémentaires
- homogénéisation du gel
- extraction de l'oxygène
- conditionnement en flacons ou seringues
- stérilisation.

12. Procédé de préparation d'un hydrogel injectable à base d'acide hyaluronique réticulé, selon l'une des revendications 1 à 10, **caractérisé en ce qu'**il comprend au moins les étapes suivantes :
- préparation d'une solution aqueuse d'acide hyaluronique sous atmosphère inerte
- réticulation de l'acide hyaluronique sous atmosphère inerte
- élimination du réticulant par dialyse
- homogénéisation du gel
- extraction de l'oxygène
- conditionnement en flacons ou seringues
- stérilisation à la chaleur humide.

13. Procédé de préparation d'un hydrogel injectable à base d'acide hyaluronique réticulé, selon l'une des revendications 1 à 10, **caractérisé en ce qu'**il comprend au moins les étapes suivantes :
- préparation d'une solution aqueuse d'acide hyaluronique
- extraction de l'oxygène
- réticulation de l'acide hyaluronique sous atmosphère inerte
- homogénéisation du gel
- extraction de l'oxygène
- conditionnement en flacons ou seringues
- stérilisation

14. Hydrogel injectable stérile contenant de l'acide hyaluronique réticulé obtenu selon l'une des revendications 1 à 13.

15. Hydrogel injectable stérile selon la revendication 14, pour une utilisation thérapeutique ou pour les applications esthétiques.

## Patentansprüche

1. Verfahren zur Herstellung eines injizierbaren Hydrogels auf der Basis von vernetzter Hyaluronsäure, oder von einem ihrer Salze, und eventuell anderer biologisch kompatibler Polymere, umfassend mindestens die folgenden Schritte:
- Herstellen eines Hydrogels, einschließend mindestens einen Vernetzungsschritt der Hyaluronsäure in wässriger Lösung, **dadurch gekennzeichnet, dass** der Vernetzungsschritt der Hyaluronsäure unter inerter Atmosphäre durchgeführt wird;
- Reinigen des Hydrogels;
- Verpacken des Hydrogels in Spritzen oder in Flakons;
- Sterilisieren;
und unbedingtes Durchführen mindestens eines Extraktionsschritts des in dem Hydrogel enthaltenen Sauerstoffs; wobei der Extraktionsschritt nach dem Vernetzungsschritt der Hyaluronsäure unter inerter Atmosphäre und vor dem und/oder während des Verpackungsschritts des Hydrogels durchgeführt wird; wobei der Extraktionsschritt darin besteht, in einem geeigneten Behälter mindestens einen Extraktionszyklus durchzuführen, der durch die folgenden aufeinanderfolgenden Phasen gekennzeichnet ist:
- Evakuieren, bei einem Druck p unter dem atmosphärischen Druck, des Behälters, der das Hydrogel enthält, während einer Dauer *t;*
- Entfernen des Vakuums am Ende der Dauer t durch Einleiten einer geeigneten Menge eines anderen Gases als Sauerstoff.

2. Verfahren nach Anspruch 1, **dadurch gekennzeichnet, dass** im Fall des Extraktionsschritts des Sauerstoffs das andere Gas als Sauerstoff ein inertes Gas oder ein Gemisch inerter Gase ist, vorzugsweise leichter als Luft.

3. Verfahren nach einem der Ansprüche 1 bis 2, **dadurch gekennzeichnet, dass** die inerte Atmosphäre aus einem oder mehreren der folgenden Gase: Wasserstoff, Helium, Stickstoff und Argon besteht.

4. Verfahren nach Anspruch 1, **dadurch gekennzeichnet, dass** die inerte Atmosphäre bei einem Druck über oder gleich dem atmosphärischen Druck ist.

5. Verfahren nach einem der Ansprüche 1 bis 4, **dadurch gekennzeichnet, dass** im Fall des Extraktionsschritts des Sauerstoffs das Evakuieren bei einem Druck unter 100 mbar, vorzugsweise unter 50 mbar, durchgeführt wird.

6. Verfahren nach einem der Ansprüche 1 bis 5, **dadurch gekennzeichnet, dass** im Fall des Extraktionsschritts des Sauerstoffs das Evakuieren dynamisch durchgeführt wird.

7. Verfahren nach einem der Ansprüche 1 bis 6, **dadurch gekennzeichnet, dass** im Fall des Extraktionsschritts des Sauerstoffs die Evakuierungsdauer zwischen 1 Minute und 60 Minuten, vorzugsweise zwischen 5 Minuten und 25 Minuten, liegt.

8. Verfahren nach einem der Ansprüche 1 bis 7, **dadurch gekennzeichnet, dass** im Fall des Extraktionsschritts des Sauerstoffs die Anzahl der Extraktionsschritte des in dem injizierbaren Hydrogel enthaltenen Sauerstoffs während seiner Herstellung zwischen 1 und 4 liegt.

9. Verfahren nach einem der Ansprüche 1 bis 8, **dadurch gekennzeichnet, dass** im Fall des Extraktionsschritts des Sauerstoffs jeder Extraktionsschritt des Sauerstoffs, der während der Herstellung des injizierbaren Hydrogels durchgeführt wird, eine Anzahl von Extraktionszyklen umfasst, die zwischen 1 und 50, vorzugsweise zwischen 2 und 10, noch vorzugsweiser zwischen 3 und 6, liegt.

10. Verfahren nach einem der Ansprüche 1 bis 9, **dadurch gekennzeichnet, dass** vor dem Vernetzungsschritt der Hyaluronsäure unter inerter Atmosphäre ein Extraktionsschritt des in dem Reaktionsgemisch enthaltenen Sauerstoffs durchgeführt wird.

11. Verfahren zur Herstellung eines injizierbaren Hydrogels auf der Basis vernetzter Hyaluronsäure nach einem der Ansprüche 1 bis 10, **dadurch gekennzeichnet, dass** es mindestens die folgenden Schritte umfasst:
- Herstellen einer wässrigen Hyaluronsäurelösung unter inerter Atmosphäre
- Vernetzen der Hyaluronsäure unter inerter Atmosphäre
- Entfernen des Vernetzungsmittels durch Reinigen
- optionales Extrahieren des Sauerstoffs
- optionales Hinzufügen komplementärer Moleküle
- Homogenisieren des Gels
- Extrahieren des Sauerstoffs
- Verpacken in Flakons oder Spritzen
- Sterilisieren.

12. Verfahren zur Herstellung eines injizierbaren Hydrogels auf der Basis vernetzter Hyaluronsäure nach einem der Ansprüche 1 bis 10, **dadurch gekennzeichnet, dass** es mindestens die folgenden Schritte umfasst:
- Herstellen einer wässrigen Hyaluronsäurelösung unter inerter Atmosphäre
- Vernetzen der Hyaluronsäure unter inerter Atmosphäre
- Entfernen des Vernetzungsmittels durch Dialyse
- Homogenisieren des Gels
- Extrahieren des Sauerstoffs
- Verpacken in Flakons oder Spritzen
- Sterilisieren bei feuchter Wärme.

13. Verfahren zur Herstellung eines injizierbaren Hydrogels auf der Basis vernetzter Hyaluronsäure nach einem der Ansprüche 1 bis 10, **dadurch gekennzeichnet, dass** es mindestens die folgenden Schritte umfasst:
- Herstellen einer wässrigen Hyaluronsäurelösung
- Extrahieren des Sauerstoffs
- Vernetzen der Hyaluronsäure unter inerter Atmosphäre
- Homogenisieren des Gels
- Extrahieren des Sauerstoffs
- Verpacken in Flakons oder Spritzen
- Sterilisieren.

14. Steriles injizierbares Hydrogel, enthaltend vernetzte Hyaluronsäure, erhalten nach einem der Ansprüche 1 bis 13.

15. Steriles injizierbares Hydrogel nach Anspruch 14 für eine therapeutische Verwendung oder für ästhetische Anwendungen.

## Claims

1. A method for preparing an injectable hydrogel based on a crosslinked hyaluronic acid, or one of the salts thereof, and optionally of other biocompatible polymers, comprising at least the following steps:
- preparing a hydrogel integrating at least one crosslinking step of the hyaluronic acid in an aqueous solution; **characterized in that** said crosslinking step of hyaluronic acid is performed in an inert atmosphere;
- purifying the hydrogel;
- packaging the hydrogel in syringes or bottles;
- sterilization,
and by performing mandatorily at least one extraction step of the oxygen contained in the hydrogel; said extraction step being performed after the crosslinking step of the hyaluronic acid in an inert atmosphere and before and/or at the time of the hydrogel packaging step; said extraction step consisting of performing at least one extraction cycle in a suitable container, **characterized by** the following successive phases:
- placing the container containing the hydrogel under a vacuum at a pressure *p* lower than atmospheric pressure for a time *t;*
- breaking the vacuum after time *t* by adding an adapted quantity of a gas other than oxygen.

2. The method according to claim 1, **characterized in that**, for the oxygen extraction step, the gas other than oxygen is an inert gas or a mixture of inert gases, preferably lighter than air.

3. The method according to one of claims 1 to 2, **characterized in that** the inert atmosphere consists of one or more of the following gases: hydrogen, helium, nitrogen and argon.

4. The method according to claim 1, **characterized in that** the inert atmosphere is at a pressure equal to or higher than atmospheric pressure.

5. The method according to one of claims 1 to 4, **characterized in that**, for the oxygen extraction step, the application of a vacuum is performed at a pressure lower than 100 mbar, preferably lower than 50 mbar.

6. The method according to one of claims 1 to 5, **characterized in that**, for the oxygen extraction step, the application of a vacuum is performed dynamically.

7. The method according to one of claims 1 to 6, **characterized in that**, for the oxygen extraction step, the time of vacuum application is between 1 minute and 60 minutes, preferably between 5 minutes and 25 minutes.

8. The method according to one of claims 1 to 7, **characterized in that**, for the oxygen extraction step, the number of steps to extract the oxygen contained in the injectable hydrogel throughout production thereof is between 1 and 4.

9. The method according to one of claims 1 to 8, **characterized in that**, for the oxygen extraction step, each oxygen extraction step performed throughout the production of the injectable hydrogel comprises a number of extraction cycles of between 1 and 50, preferably between 2 and 10, further preferably between 3 and 6.

10. The method according to one of claims 1 to 9, **characterized in that**, before the crosslinking step of the hyaluronic acid in an inert atmosphere, a step to extract the oxygen contained in the reaction medium is performed.

11. The method for preparing an injectable hydrogel based on a crosslinked hyaluronic acid, according to one of claims 1 to 10, **characterized in that** it comprises at least the following steps:
- preparing an aqueous solution of hyaluronic acid in an inert atmosphere
- crosslinking the hyaluronic acid in an inert atmosphere
- removing the crosslinker by purification
- optionally extracting oxygen
- optionally adding additional molecules
- homogenizing the gel
- extracting oxygen
- packaging in bottles or syringes
- sterilization.

12. The method for preparing an injectable hydrogel based on a crosslinked hyaluronic acid, according to one of claims 1 to 10, **characterized in that** it comprises at least the following steps:
- preparing an aqueous solution of hyaluronic acid in an inert atmosphere
- crosslinking the hyaluronic acid in an inert atmosphere
- removing the crosslinker via dialysis
- homogenizing the gel
- extracting oxygen
- packaging in bottles or syringes
- sterilization under moist heat.

13. The method for preparing an injectable hydrogel based on a crosslinked hyaluronic acid, according to one of claims 1 to 10, **characterized in that** it comprises at least the following steps:
- preparing an aqueous solution of hyaluronic acid
- extracting oxygen
- crosslinking the hyaluronic acid in an inert atmosphere
- homogenizing the gel
- extracting oxygen
- packaging in bottles or syringes
- sterilization.

14. A sterile injectable hydrogel containing crosslinked hyaluronic acid obtained according to one of claims 1 to 13.

15. The sterile injectable hydrogel according to claim 14, for use in therapeutic treatment or for cosmetic applications.
